(19) Europäisches Patentamt
European Patent Office
Office européen des brevets

(11) Veröffentlichungsnummer: **0 203 606 B1**

(12) # EUROPÄISCHE PATENTSCHRIFT

(45) Veröffentlichungstag der Patentschrift: **31.10.90**

(51) Int. Cl.⁵: **C07C 69/734**, C07C 205/06, A01N 37/10, C07C 271/60

(21) Anmeldenummer: **86107326.0**

(22) Anmeldetag: **30.05.86**

(54) **Stilbenderivate und Fungizide, die diese Verbindungen enthalten.**

(30) Priorität: **30.05.85 DE 3519280**

(43) Veröffentlichungstag der Anmeldung: **03.12.86 Patentblatt 86/49**

(45) Bekanntmachung des Hinweises auf die Patenterteilung: **31.10.90 Patentblatt 90/44**

(84) Benannte Vertragsstaaten: **AT BE CH DE FR GB IT LI NL SE**

(56) Entgegenhaltungen: **EP-A- 0 178 826**

(73) Patentinhaber: **BASF Aktiengesellschaft, Carl-Bosch-Strasse 38, D-6700 Ludwigshafen(DE)**

(72) Erfinder: **Schirmer, Ulrich, Dr., Berghalde 79, D-6900 Heidelberg(DE)**
Erfinder: **Karbach, Stefan, Dr., Sperlinggasse 3, D-6700 Ludwigshafen(DE)**
Erfinder: **Pommer, Ernst-Heinrich, Dr., Berliner Platz 7, D-6703 Limburgerhof(DE)**
Erfinder: **Ammermann, Eberhard, Dr., Sachsenstrasse 3, D-6700 Ludwigshafen(DE)**
Erfinder: **Steglich, Wolfgang, Prof. Dr., Hobsweg 77, D-5300 Bonn-Roettgen(DE)**
Erfinder: **Schwalge, Barbara Agnes Maria, Tannenweg 9, D-5204 Lohmar 1(DE)**
Erfinder: **Anke, Timm, Prof. Dr., Theodor-Heuss-Strasse 17, D-6750 Kaiserslautern(DE)**

EP 0 203 606 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Stilbenderivate und Fungizide, welche diese Verbindungen enthalten.

Es ist bekannt, N-Trichlormethylthio-tetrahydrophthalimid als Fungizid in der Landwirtschaft sowie im Obst- und Gartenbau einzusetzen (Chem. Week, June 21, 1972, Seite 46). Das bekannte Mittel ist jedoch nur vor der Infektion verwendbar und seine Wirkung genügt bei niedrigen Aufwandmengen den Anforderungen der Praxis nicht.

Es wurde nun gefunden, daß neue Stilbenderivate der Formel

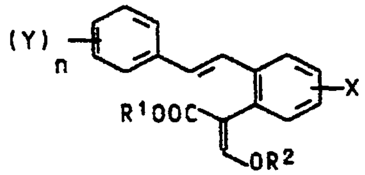

in der $R^1$ und $R^2$ Methyl bedeuten,
X Wasserstoff, Halogen, $C_1$–$C_4$ Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, $NO_2$, Alkylthio, Thiocyanato, Cyano, Aralkyloxy, Aryloxymethyl,

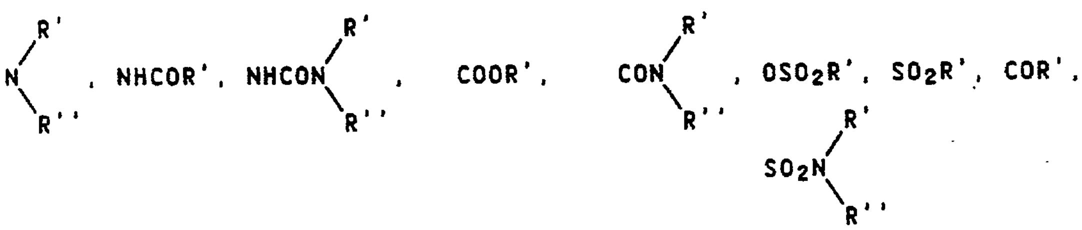

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen 2-(β-Methoxy-α-methoxycarbonylvinyl)-stilben, 2-(β-Methoxy-α-methoxycarbonylvinyl)-4′-chlor-stilben, 2-(β-Methoxy-α-methoxycarbonylvinyl)-4′-fluorstilben und 2-(β-Methoxy-α-methoxycarbonylvinyl)-2′,6′-dichlor-stilben,
eine ausgezeichnete fungizide Wirkung haben.

Die in der allgemeinen Formel angeführten Reste können beispielsweise folgende Bedeutung haben:
X Wasserstoff, Halogen (z.B. Fluor, Chlor, Brom), $C_1$–$C_4$-Alkoxy (z.B. Methoxy, n-Butoxy), Trifluormethyl, Cyano oder $NO_2$,
Y Wasserstoff, $C_1$–$C_{12}$-Alkyl (z.B. Methyl, Ethyl, t-Butyl, Dodecyl), Halogen-$C_1$–$C_4$-Alkoxy-$C_1$–$C_4$-alkyl (z.B. Methoxymethyl), $C_5$–$C_8$-Cycloalkyl (z.B. Cyclohexyl), Aralkyl (z.B. Benzyl, Phenethyl), Aryl (z.B. Phenyl), Aryloxy (z.B. Phenoxy), Halogen (z.B. Fluor, Chlor, Brom oder Jod) eine gegebenenfalls substituierte $C_4H_4$-Kette, die mit dem Benzolring zu einem gegebenenfalls substituierten Naphthylring kondensiert ist, $C_1$–$C_6$-Alkoxy (z.B. Isopropoxy, Hexyloxy), Halogen-$C_1$–$C_4$-alkoxy (z.B. 1,1,2,2-Tetrafluorethoxy), $C_1$–$C_4$-Alkylthio (z.B. Methylthio), Thiocyanato, Cyano, $NO_2$, Aralkyloxy (Benzyloxy, Phenethyloxy), Aryloxymethyl (Phenoxymethyl),

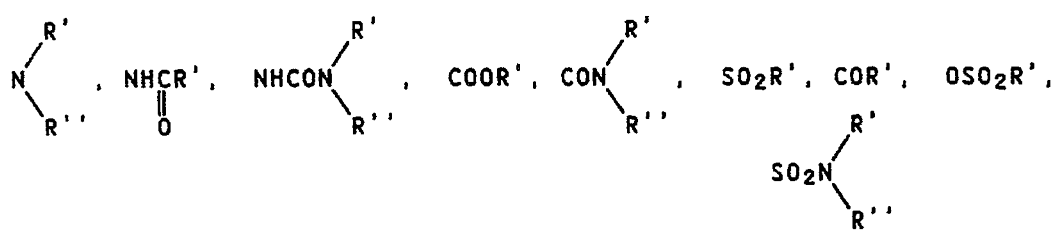

wobei R′ und R″ jeweils unabhängig voneinander Wasserstoff, $C_1$–$C_4$-Alkyl (z.B. Methyl, Ethyl), $C_1$–$C_4$-Alkoxy (z.B. Methoxy, tert.-Butoxy), $C_1$–$C_4$-Alkylthio (z.B. Methylthio), $C_5$–$C_8$-Cycloalkyl (z.B. Cyclohexyl) oder gegebenenfalls durch $C_1$–$C_4$-Alkyl, Halogen oder $C_1$–$C_4$-Alkoxy substituiertes Phenyl (z.B. Phenyl, 3-Chlorphenyl, 4-Methylphenyl, 3-Methoxyphenyl) bedeuten.

Die neuen fungiziden Stilbenderivate können als E- oder Z-Isomere an den beiden Doppelbindungen vorliegen. Die Stereoisomeren lassen sich beispielsweise durch Säulenchromatographie trennen oder aufgrund von Löslichkeitsunterschieden in reiner Form isolieren. Die isomerenreinen Verbindungen lassen sich durch bekannte Methoden in die jeweils anderen Isomere überführen. Die isomerenreinen Verbindungen werden wie ihre Mischungen von der vorliegenden Erfindung umfaßt. Für die Anwendung der neuen Verbindungen als Fungizide sind sowohl die Diastereomerengemische als auch die einheitlichen isomeren Verbindungen wie auch deren bei der Synthese anfallenden Gemische geeignet.

Die neuen Verbindungen können beispielsweise nach folgendem Verfahren hergestellt werden:

2-Methylphenylessigsäureester der allgemeinen Formel

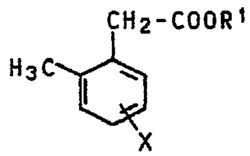

werden nach Wislicenus (Liebigs Annalen 424, 215 (1921) und Liebigs Annalen 413, 206 (1917)) mit Ameisensäuremethylester und Natriumhydrid in einem inerten Lösungsmittel zur Reaktion gebracht. Die so erhaltenen Verbindungen der allgemeinen Formel

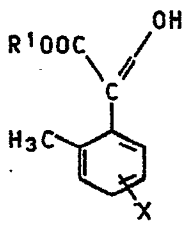

werden mit einem Alkylierungsmittel in Gegenwart einer Base in einem Lösungsmittel (z.B. Aceton) zu α-(2-Methylphenyl)-β-alkoxyacrylsäureestern umgesetzt,

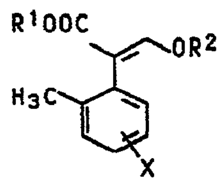

in denen $R^1$, $R^2$ und X die oben genannten Bedeutungen haben.

Die Bromierung dieser Verbindung mit N-Bromsuccinimid (Horner, Winkelmann, Angew. Chemie 71, 349 (1959) führt zu α-(2-Brommethylphenyl)-β-alkoxy-acrylsäureestern, die mit Phosphorigsäuretrialkylestern zu Phosphonsäureestern der allgemeinen Formel reagieren:

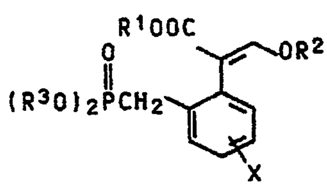

in der $R^1$, $R^2$ und X die oben genannten Bedeutungen haben und $R^3$ für $C_1$–$C_8$-Alkyl steht (Houben-Weyl, Methoden der organischen Chemie 12/1, 433 ff (1963)).

Die oben genannten Phosphonsäureester werden mit gegebenenfalls substituierten Benzaldehyden zu den neuen Stilbenderivaten umgesetzt:

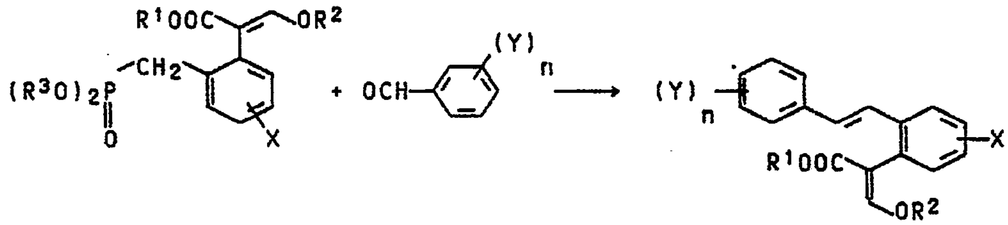

(vgl. Wadsworth, Emmons, JACS, 83, 1732 (1961)).

Die nachstehenden Beispiele erläutern die Synthese der neuen Verbindungen:

Vorschrift A

α-(2-Methylphenyl)-β-methoxy-acrylsäuremethylester

16,5 g 2-Methylphenylessigsäuremethylester werden in 10 ml Ameisensäuremethylester gelöst und langsam zu einer Suspension aus 3 g Natriumhydrid in 150 ml abs. Ether getropft. Nach 4 h Rückfluß wird mit verdünnter HCl angesäuert, die org. Phase abgetrennt, mit Wasser gewaschen, über $MgSO_4$ getrocknet und eingeengt. Man erhält 13,8 g eines hellgelben Öles (α-Formyl-(2-methylphenyl)essigsäuremethylester), das zusammen mit 5,8 ml Dimethylsulfat, 10,9 g Kaliumcarbonat und 70 ml Aceton 1 h am Rückfluß gekocht wird. Nach dem Filtrieren und Einengen wird in Ether aufgenommen, dann mit verdünntem wäßrigem Ammoniak und mehrfach mit Wasser gewaschen. Nach Abziehen des Ethers erhält man 11,3 g rohen α-(2-Methylphenyl)-β-methoxyacrylsäuremethylester ($Kp_{0,05}$ 102–108°C).

| | | | |
|---|---|---|---|
| NMR in $CDCl_3$: | 7,53 | s | 1H |
| | 7,16-7,36 | bs | 4H |
| | 3,64 | s | 3H |
| | 3,73 | s | 3H |
| | 2,16 | s | 3H |

Vorschrift B

α-(2-Brommethylphenyl)-β-methoxyacrylsäuremethylester

20,6 g des nach Vorschrift A erhaltenen α-(2-Methylphenyl)-β-methoxyacrylsäuremethylesters, 17,65 g N-Bromsuccinimid, 0,2 g Azodiisobutyronitril und 150 ml $CCl_4$ werden langsam auf 90°C erhitzt. Man hält bei dieser Temperatur, bis alles Succinimid auf dem Lösungsmittel schwimmt.

Nach Filtration wird eingeengt, das verbleibende Öl in etwa 5 ml Aceton gelöst und mit n-Hexan zur Kristallisation gebracht. Man erhält 27,5 g farblose Kristalle vom Fp. 86–87°C.

Vorschrift C

2-(β-Methoxy-α-methoxycarbonyl-vinyl)benzylphosphonsäuredimethylester

28,5 g α-(2-Brommethylphenyl)-β-methoxyacrylsäuremethylester werden mit 11,8 ml Phosphorigsäuretrimethylester und 6,5 ml Toluol eine Stunde am Rückfluß gekocht. Das Reaktionsgemisch wird vorsichtig im Vakuum eingeengt, das verbleibende Öl wird nach Lösen in 5 ml Ether mit n-Hexan zur Kristallisation gebracht. Man erhält 27,3 g farblose Kristalle vom Fp. 95–97°C.

In entsprechender Weise lassen sich folgende Verbindungen herstellen:

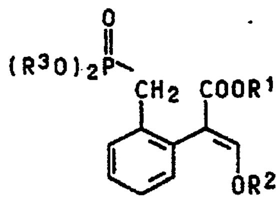

| $R^1$ | $R^2$ | $R^3$ | Fp° C/NMR |
|---|---|---|---|
| $CH_3$ | $CH_3$ | $C_2H_5$ | Harz 53-56 |
| $C_2H_5$ | $CH_3$ | $CH_3$ | Harz |
| $i-C_3H_7$ | $CH_3$ | $CH_3$ | Harz |
| $n-C_6H_{13}$ | $CH_3$ | $CH_3$ | |
| $CH_3$ | $C_2H_5$ | $CH_3$ | 103-104 |
| $CH_3$ | $i-C_3H_7$ | $CH_3$ | |
| $CH_3$ | $n-C_6H_{13}$ | $CH_3$ | |
| $CH_3$ | $s-C_4H_9$ | $CH_3$ | |
| $n-C_4H_9$ | $CH_3$ | $CH_3$ | Harz |
| $n-C_3H_7$ | $CH_3$ | $CH_3$ | |
| $s-C_4H_9$ | $CH_3$ | $CH_3$ | |
| $CH_3$ | $n-C_3H_7$ | $CH_3$ | |
| $CH_3$ | $CH_3$ | $C_4H_9$ | |
| $CH_3$ | $CH_3$ | $i-C_3H_7$ | |
| $CH_3$ | $CH_3$ | $n-C_6H_{13}$ | Öl |

In der oben genannten Weise lassen sich folgende Verbindungen herstellen:

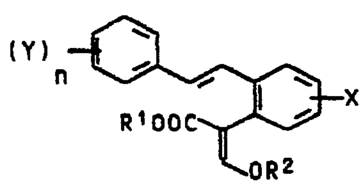

| Nr. | $R^1$ | $R^2$ | X | $(Y)_n$ | Fp °C/NMR |
|---|---|---|---|---|---|
| 2 | $C_2H_5$ | $CH_3$ | H | H | |
| 3 | i-$C_3H_7$ | $CH_3$ | H | H | |
| 4 | $nC_6H_{13}$ | $CH_3$ | H | H | |
| 5 | n-$C_4H_9$ | $CH_3$ | H | H | |
| 6 | n-$C_3H_7$ | $CH_3$ | H | H | |
| 7 | s-$C_4H_9$ | $CH_3$ | H | H | |
| 8 | $CH_3$ | $C_2H_5$ | H | H | |
| 9 | $CH_3$ | i-$C_3H_7$ | H | H | |
| 10 | $CH_3$ | n-$C_6H_{13}$ | H | H | |
| 11 | $CH_3$ | n-$C_3H_7$ | H | H | |
| 12 | $CH_3$ | s-$C_4H_9$ | H | H | |
| 13 | $CH_3$ | $CH_3$ | H | H | |
| 14 | $CH_3$ | $CH_3$ | 3-Cl | H | |
| 15 | $CH_3$ | $CH_3$ | 4-Cl | H | |
| 16 | $CH_3$ | $CH_3$ | 5-Cl | H | |
| 17 | $CH_3$ | $CH_3$ | 6-Cl | H | |
| 18 | $CH_3$ | $CH_3$ | 5-$OCH_3$ | H | |
| 19 | $CH_3$ | $CH_3$ | 5-$CF_3$ | H | |
| 20 | $CH_3$ | $CH_3$ | 5-CN | H | |
| 21 | $CH_3$ | $CH_3$ | 5-$NO_2$ | 4-$CH_3$ | |
| 22 | $CH_3$ | $CH_3$ | 6-$NO_2$ | H | |
| 23 | $CH_3$ | $CH_3$ | H | 4-t-$C_4H_9$ | |
| 24 | $CH_3$ | $CH_3$ | H | 4-$C_2H_5$ | |
| 25 | $CH_3$ | $CH_3$ | H | 4-$CH_3$ | 138-139 |
| 26 | $CH_3$ | $CH_3$ | H | 2-$CH_3$ | 156-158 |
| 27 | $CH_3$ | $CH_3$ | H | 3-$CH_3$ | 95-97 |
| 28 | $CH_3$ | $CH_3$ | H | 2-Cl | 147-148 |
| 29 | $CH_3$ | $CH_3$ | H | 3-Cl | 110-111 |
| 31 | $CH_3$ | $CH_3$ | H | 2,4-$Cl_2$ | 134-136 |
| 32 | $CH_3$ | $CH_3$ | H | 3,5-$Cl_2$ | 144-147 |
| 33 | $CH_3$ | $CH_3$ | H | 2-F | 69-70 |
| 34 | $CH_3$ | $CH_3$ | H | 3-F | 103-105 |
| 36 | $CH_3$ | $CH_3$ | H | 3-$CF_3$ | 75-77 |

| Nr. | $R^1$ | $R^2$ | X | $(Y)_n$ | Fp °C/NMR |
|---|---|---|---|---|---|
| 37 | $CH_3$ | $CH_3$ | H | $4-CF_3$ | 124-125 |
| 38 | $CH_3$ | $CH_3$ | H | 4-Br | 138-139 |
| 39 | $CH_3$ | $CH_3$ | H | 3-Phenoxy | 85-87 |
| 40 | $CH_3$ | $CH_3$ | H | 4-Phenoxy | |
| 41 | $CH_3$ | $CH_3$ | H | $2-OCH_3$ | 60-62 |
| 42 | $CH_3$ | $CH_3$ | H | $3-OCH_3$ | 102-103 |
| 43 | $CH_3$ | $CH_3$ | H | $4-OCH_3$ | 125-127 |
| 44 | $CH_3$ | $CH_3$ | H | $4-O(t)-C_4H_9$ | |
| 45 | $CH_3$ | $CH_3$ | H | $4-O(n)-C_4H_9$ | |
| 46 | $CH_3$ | $CH_3$ | H | $4-CH_2OCH_3$ | |
| 47 | $CH_3$ | $CH_3$ | H | 4-J | |
| 48 | $CH_3$ | $CH_3$ | H | 2,3 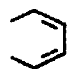 | Harz |
| 49 | $CH_3$ | $CH_3$ | H | 3,4 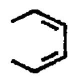 | 158-159 |
| 50 | $CH_3$ | $CH_3$ | H | $4-OCHF_2$ | |
| 51 | $CH_3$ | $CH_3$ | H | $3-OCF_2CHF_2$ | |
| 52 | $CH_3$ | $CH_3$ | H | $4-SCH_3$ | |
| 53 | $CH_3$ | $CH_3$ | H | 4-CN | |
| 54 | $CH_3$ | $CH_3$ | H | 3-CN | |
| 55 | $CH_3$ | $CH_3$ | H | 4-SCN | |
| 56 | $CH_3$ | $CH_3$ | H | $4-N(CH_3)_2$ | |
| 57 | $CH_3$ | $CH_3$ | H | $3-NHCOCH_3$ | |
| 58 | $CH_3$ | $CH_3$ | H | $3-NHCOOCH_3$ | |
| 59 | $CH_3$ | $CH_3$ | H | $4-NHCON(CH_3)_2$ | |
| 60 | $CH_3$ | $CH_3$ | H | $4-COOCH_3$ | |
| 61 | $CH_3$ | $CH_3$ | H | $4-CONHCH_3$ | |
| 62 | $CH_3$ | $CH_3$ | H | $4-SO_2CH_3$ | |
| 63 | $CH_3$ | $CH_3$ | H | 4-Phenylsulfonyl | |
| 64 | $CH_3$ | $CH_3$ | H | $3-COCH_3$ | |
| 65 | $CH_3$ | $CH_3$ | H | $4-OSO_2CH_3$ | |
| 66 | $CH_3$ | $CH_3$ | H | $4-SO_2N(CH_3)_2$ | |
| 67 | $CH_3$ | $CH_3$ | H | 4-NHCONH 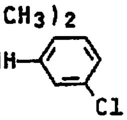 | |
| 68 | $CH_3$ | $CH_3$ | H | 4-Benzoyl | |

| Nr. | $R^1$ | $R^2$ | X | $(Y)_n$ | Fp °C/NMR |
|---|---|---|---|---|---|
| 69 | $CH_3$ | $CH_3$ | H | 3-$NO_2$ | |
| 70 | $CH_3$ | $CH_3$ | H | 4-$NO_2$ | |
| 71 | $CH_3$ | $CH_3$ | H | 2-Cl-6F | |
| 72 | $CH_3$ | $CH_3$ | H | 2,4,5$(CH_3)_3$ | |
| 73 | $CH_3$ | $CH_3$ | H | 3,4,5$(OCH_3)_3$ | 118-121 |
| 74 | $CH_3$ | $CH_3$ | H | 2,4$(CH_3)_2$ | |
| 75 | $CH_3$ | $CH_3$ | H | 4-i-$C_3H_7$ | |
| 76 | $CH_3$ | $CH_3$ | H | 4-Phenyl | |
| 77 | $CH_3$ | $CH_3$ | H | 2,3,4-$Cl_3$ | |
| 79 | $CH_3$ | $CH_3$ | H | 3,4-$Cl_2$ | 96-98 |
| 80 | $CH_3$ | $CH_3$ | H | 3-$NO_2$4$CH_3$ | |
| 81 | $CH_3$ | $CH_3$ | H | 4-$N(C_2H_5)_2$ | |
| 82 | $CH_3$ | $CH_3$ | H | 2,4,5$(OCH_3)_3$ | |
| 83 | $CH_3$ | $CH_3$ | H | 3,5-$(OCH_3)_2$ | |
| 84 | $CH_3$ | $CH_3$ | H | 3-Benzyloxy | 77-70 |
| 85 | $CH_3$ | $CH_3$ | H | 2,4,6$(OCH_3)_3$ | |
| 86 | $CH_3$ | $CH_3$ | H | 4-O(n)$C_6H_{13}$ | |
| 87 | $CH_3$ | $CH_3$ | H | 2-Cl5$NO_2$ | |
| 88 | $CH_3$ | $CH_3$ | H | 3$NO_2$4Cl | 168-169 |
| 89 | $CH_3$ | $CH_3$ | H | 2-Cl6$NO_2$ | |
| 90 | $CH_3$ | $CH_3$ | H | 2-$OCF_2CHF_2$ | |
| 91 | $CH_3$ | $CH_3$ | H | 3-Br4$OCH_3$ | |
| 92 | $C_2H_5$ | $CH_3$ | H | 3-Cl | |
| 93 | $C_2H_5$ | $CH_3$ | H | 4-Cl | |
| 94 | $C_2H_5$ | $CH_3$ | H | 3,5$Cl_2$ | |
| 95 | $C_2H_5$ | $CH_3$ | H | 4-F | |
| 96 | $C_2H_5$ | $CH_3$ | H | 4-Br | |
| 97 | $C_2H_5$ | $CH_3$ | H | 4-$CH_3$ | |
| 98 | $C_2H_5$ | $CH_3$ | H | 3,4-$(CH_3)_2$ | |
| 99 | $C_2H_5$ | $CH_3$ | H | 4-$OCH_3$ | |
| 100 | $C_2H_5$ | $CH_3$ | H | 3,4,5-$(OCH_3)_3$ | |

| Nr. | $R^1$ | $R^2$ | X | $(Y)_n$ | Fp °C/NMR |
|---|---|---|---|---|---|
| 101 | $C_2H_5$ | $CH_3$ | H | $3-CH_3$ | |
| 102 | $C_2H_5$ | $CH_3$ | H | $4-(t)C_4H_9$ | |
| 103 | $C_2H_5$ | $CH_3$ | H | $2CH_3$ | |
| 104 | $CH_3$ | $C_2H_5$ | H | 4-Cl | |
| 105 | $CH_3$ | $C_2H_5$ | H | 4-F | |
| 106 | $CH_3$ | $C_2H_5$ | H | $4-CH_3$ | |
| 107 | $CH_3$ | $C_2H_5$ | H | $4-OCH_3$ | |
| 108 | $CH_3$ | $C_2H_5$ | H | $4-NO_2$ | |
| 109 | $CH_3$ | $CH_3$ | H | 4-Benzyloxy | |
| 110 | $CH_3$ | $CH_3$ | H | 3-Phenethyl | |
| 111 | $CH_3$ | $CH_3$ | H | 4-Phenethyl | |
| 112 | $CH_3$ | $CH_3$ | H | 3-Phenethyloxy | |
| 113 | $CH_3$ | $CH_3$ | H | 4-Phenethyloxy | |
| 114 | $CH_3$ | $CH_3$ | H | 3-Phenoxymethyl | |
| 115 | $CH_3$ | $CH_3$ | H | 4-Phenoxymethyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der

wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,05 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für die folgenden Versuche wurde als Vergleich der bekannte Wirkstoff N-Trichlormethylthiotetrahydrophthalimid (A) verwendet.

Anwendungsbeispiel 1

Wirksamkeit gegen Phytophthora infestans an Tomaten

Blätter von Topfpflanzen der Sorte "Große Fleischtomate" werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß die Wirkstoffe Nr. 25, 26, 27, 28, 29, 34, 37, 38, 39, 41, 48, 49, 73, 79 und 84 bei der Anwendung als 0,025%ige (Gew.%) Spritzbrühe eine bessere fungizide Wirkung haben (95%) als der bekannte Wirkstoff A (80%).

Anwendungsbeispiel 2

Wirksamkeit gegen Plasmopara viticola

Blätter von Topfreben der Sorte "Müller-Thurgau" werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pfanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß die Wirkstoffe Nr. 26, 27, 29, 32, 33, 34, 37, 38, 39, 42, 49, 79, 84 und 88 bei der Anwendung als 0,05; 0,0125 und 0,006%ige Spritzbrühe eine gute fungizide Wirkung haben (100%).

Anwendungsbeispiel 5

Wirksamkeit gegen Pyrenophora teres

Gerstenkeimlinge der Sorte "Asse" werden im Zweiblattstadium mit wäßrigen Suspensionen, die 80% Wirkstoff und 20% Emulgator in der Trockensubstanz enthalten, tropfnaß gespritzt. Nach 24 Stunden werden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend werden die Pflanzen im Gewächshaus bei 20 bis 22°C und 70% relativer Luftfeuchtigkeit für weitere 5 Tage kultiviert. Dann wird das Ausmaß der Symptomentwicklung ermittelt.

Das Ergebnis des Versuches zeigt, daß die Wirkstoffe Nr. 25, 26, 27, 28, 29, 33, 34, 36, 39, 41, 49, 73, 79, 84 und 88 bei der Anwendung als 0,05%ige Spritzbrühe eine gute fungizide Wirkung (97%) zeigen.

Anwendungsbeispiel 6

Wirksamkeit gegen Weizenmehltau

Blätter von in Töpfen gewachsenen Weizenkeimlingen der Sorte Frühgold werden mit wäßriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 7 Tagen wird das Ausmaß der Mehltauentwicklung ermittelt.

Das Ergebnis des Versuches zeigt, daß die Wirkstoffe Nr. 25, 26, 27, 28, 29, 32, 33, 34, 36 und 37 bei der Anwendung als 0,025 und 0,006%ige Spritzbrühe eine gute fungizide Wirkung zeigen (97%).

## Patentansprüche

1. Stilbenderivate der allgemeinen Formel

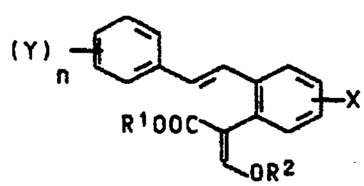

in der $R^1$ und $R^2$ Methyl bedeuten,
X Wasserstoff, Halogen, $C_1$–$C_4$-Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$, Aralkyloxy, Aryloxymethyl

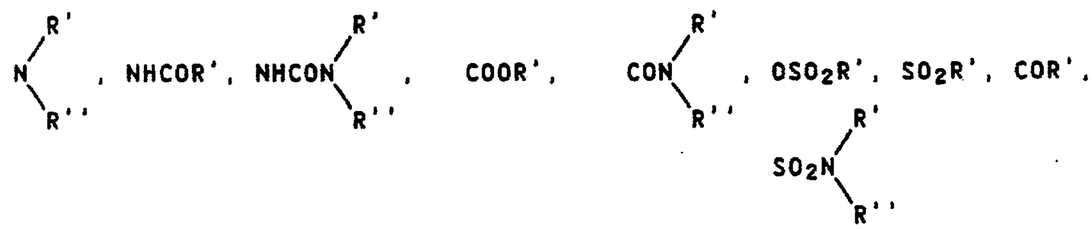

bedeutet, wobei R′ und R″ jeweils unabhängig voneinander Wassserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen 2-(β-Methoxy-α-methoxycarbonylvinyl)-stilben, 2-(β-Methoxy-α-methoxycarbonylvinyl)-4′-chlor-stilben, 2-(β-Methoxy-α-methoxycarbonylvinyl)-4′-fluor-stilben und 2-(β-Methoxy-α-methoxycarbonylvinyl)-2′,6′-dichlor-stilben.

2. Fungizid, enthaltend eine Verbindung gemäß Anspruch 1.

3. Verfahren zur Bekämpfung von Pilzen, dadurch gekennzeichnet, daß man die Pilze oder die von Pilzbefall bedrohten Materialien, Pflanzen, Saatgüter oder den Boden mit einer fungizid wirksamen Mengen einer Verbindung gemäß Anspruch 1 behandelt.

4. Fungizid, enthaltend einen festen oder flüssigen Trägerstoff und ein Stilbenderivat der allgemeinen Formel

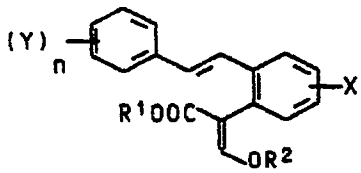

in der $R^1$ und $R^2$ Methyl bedeuten,
X Wasserstoff, Halogen, $C_1$–$C_4$-Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkyloxy, Halogenalkoxy, Alkylthio, Thiocyanato, Cyano, $NO_2$, Aralkyloxy, Aryloxymethyl

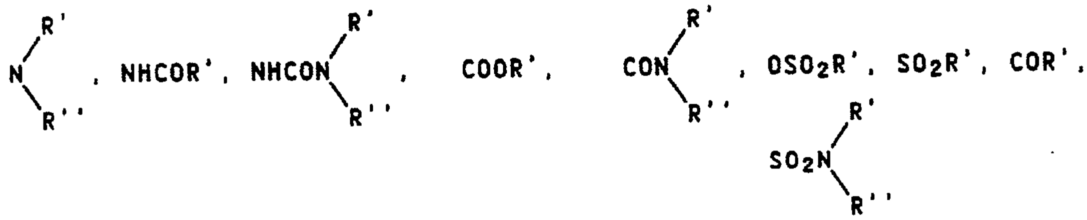

$NHCOR'$, $COOR'$, $OSO_2R'$, $SO_2R'$, $COR'$,

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen 2-(β-Methoxy-α-methoxycarbonylvinyl)-stilben, 2-(β-Methoxy-α-methoxycarbonylvinyl)-4′-chlor-stilben, 2-(β-Methoxy-α-methoxycarbonylvinyl)-4′-fluor-stilben und 2-(β-Methoxy-α-methoxycarbonylvinyl)-2′,6′-dichlor-stilben.

5. 2-(β-Methoxy-α-methoxycarbonylvinyl)-2′-methyl-stilben.

6. 2-(β-Methoxy-α-methoxycarbonylvinyl)-2′-chlor-stilben.

## Claims

1. A stilbene derivative of the formula

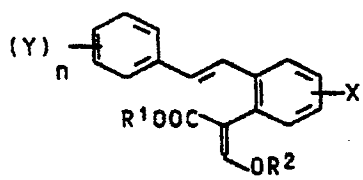

where $R^1$ and $R^2$ are each methyl, X is hydrogen, halogen, $C_1$–$C_4$alkoxy, trifluoromethyl, cyano or nitro, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$, aralkyloxy, aryloxymethyl

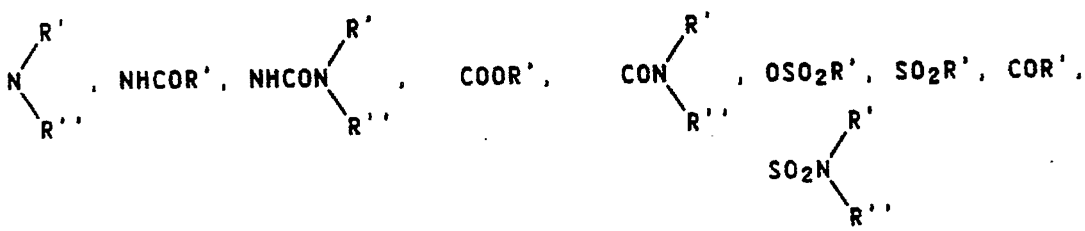

$NHCOR'$, $COOR'$, $OSO_2R'$, $SO_2R'$, $COR'$,

and R′ and R″ independently of one another are each hydrogen, alkyl, alkoxy, alkylthio or cycloalkyl or are each phenyl which is unsubstituted or substituted by alkyl, halogen or alkoxy, and n is from 1 to 4,

with the exception of the compounds 2-(β-methoxy-α-methoxycarbonylvinyl)-stilbene, 2-(β-methoxy-α-methoxycarbonylvinyl)-4′-chlorostilbene, 2-(β-methoxy-α-methoxycarbonylvinyl)-4′-fluorostilbene and 2-(β-methoxy-α-methoxy-carbonylvinyl)-2′,6′-dichlorostilbene.

2. A fungicide containing a compound as claimed in claim 1.

3. A method of controlling fungi, wherein the fungi or the materials, plants, seed or soil threatened by fungal attack is or are treated with a fungicidally effective amount of a compound as claimed in claim 1.

4. A fungicide containing a solid or liquid carrier and a stilbene derivative of the formula

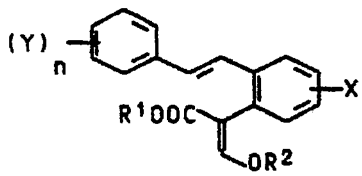

where $R^1$ and $R^2$ are each methyl, X is hydrogen, halogen, $C_1$–$C_4$alkoxy, trifluoromethyl, cyano or nitro, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$, aralkyloxy, aryloxymethyl

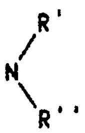 , NHCOR', 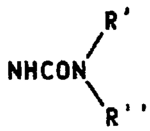 , COOR', 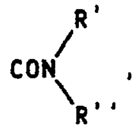 , $OSO_2R'$, $SO_2R'$, COR', 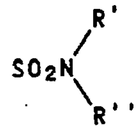

and R' and R" independently of one another are each hydrogen, alkyl, alkoxy, alkylthio or cycloalkyl or are each phenyl which is unsubstituted or substituted by alkyl, halogen or alkoxy, and n is from 1 to 4, with the exception of the compounds 2-(β-methoxy-α-methoxycarbonylvinyl)-stilbene, 2-(β-methoxy-α-methoxycarbonylvinyl)-4′-chlorostilbene, 2-(β-methoxy-α-methoxycarbonylvinyl)-4′-fluorostilbene and 2-(β-methoxy-α-methoxy-carbonylvinyl)-2′,6′-dichlorostilbene.

5. 2-(β-Methoxy-α-methoxycarbonylvinyl)-2′-methylstilbene.

6. 2-(β-Methoxy-α-methoxycarbonylvinyl)-2′-chlorostilbene.

## Revendications

1. Dérivés de stilbène de la formule générale

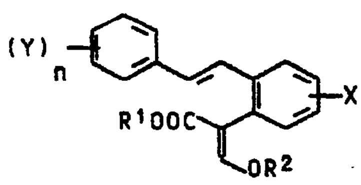

dans laquelle
$R^1$ et $R^2$ représentent méthyle,
X représente hydrogène, halogène, alcoxy en $C_1$–$C_4$, trifluorométhyle, cyano ou nitro,
Y signifie hydrogène, alkyle, halogenalkyle, alcoxyalkyle, cycloalkyle, aralkyle, aryle, aryloxy, halogène, une chaîne $C_4$–$H_4$ condensée, éventuellement substituée sur le groupe benzène, alcoxy, halogenalcoxy, alkylthio, thiocyanato, cyano, $NO_2$, aralkyloxy, aryloxyméthyle,

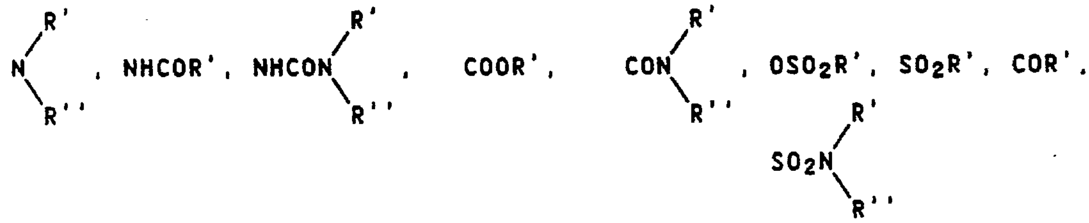
, NHCOR', COOR', OSO2R', SO2R', COR',

R' et R'' signifiant chacun, indépendamment l'un de l'autre, hydrogène, alkyle, alcoxy, alkylthio, cycloalkyle ou phényle éventuellement substitué par alkyle, halogène ou alcoxy, et
n signifie un nombre de 1 à 4, à l'exception des composés 2-(β-méthoxy-α-méthoxycarbonylvinyl)-stilbène, 2-(β-méthoxy-α-méthoxycarbonylvinyl)-4'-chlorostilbène, 2-(β-méthoxy-α-méthoxy-carbonylvinyl)-4'-fluorostilbène et 2-(β-méthoxy-α-méthoxycarbonyl-vinyl)-2',6'-dichlorostilbène.

2. Fongicide, contenant un composé selon la revendication 1.

3. Procédé de lutte contre les champignons, caractérisé par le fait que l'on traite les champignons ou les matériaux, plantes, semences ou le sol menacés d'une attaque par champignons avec une quantité efficace du point de vue herbicide d'un composé selon la revendication 1.

4. Fongicide contenant un support solide ou liquide et un dérivé de stilbène de la formule générale

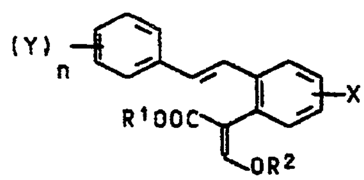

dans laquelle
$R^1$ et $R^2$ représentent méthyle,
X représente hydrogène, halogène, alcoxy en $C_1$–$C_4$, trifluorométhyle, cyano ou nitro,
Y signifie hydrogène, alkyle, halogenalkyle, alcoxyalkyle, cycloalkyle, aralkyle, aryloxy, halogène, une chaîne $C_4$–$H_4$ condensée, éventuellement substituée sur le groupe benzène, alcoxy, halogenalcoxy, alkylthio, thiocyanato, cyano, $NO_2$, aralkyloxy, aryloxyméthyle,

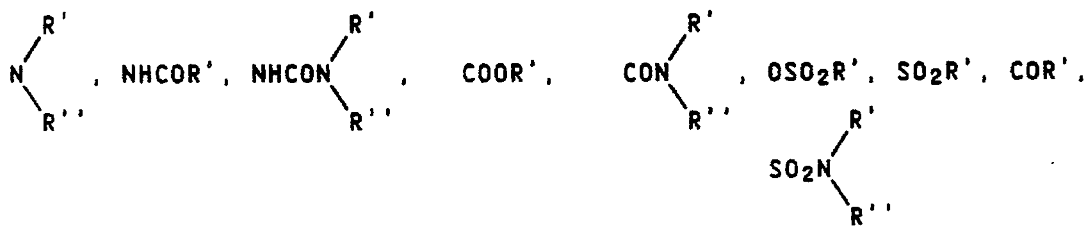
, NHCOR', COOR', OSO2R', SO2R', COR',

R' et R'' signifiant chacun, indépendamment l'un de l'autre, hydrogène, alkyle, alcoxy, alkylthio, cycloalkyle ou phényle éventuellement substitué par alkyle, halogène ou alcoxy, et
n signifie un nombre de 1 à 4, à l'exception des composés 2-(β-méthoxy-α-méthoxycarbonylvinyl)-4'-chlorostilbène, 2-(β-méthoxy-α-méthoxy-carbonylvinyl)-4'-fluorostilbène et 2-(β-méthoxy-α-méthoxy-carbonyl-vinyl)-2',6'-dichlorostilbène.

5. 2-(β-méthoxy-α-méthoxycarbonylvinyl)-2'-méthyl-stilbène.

6. 2-(β-méthoxy-α-méthoxycarbonylvinyl)-2'-chlorostilbène.